# EUROPÄISCHE PATENTSCHRIFT

(11) **EP 0 989 867 B1**
(45) Veröffentlichungstag und Bekanntmachung des Hinweises auf die Patenterteilung: **24.04.2002**
(21) Anmeldenummer: 98941248.1
(22) Anmeldetag: 29.06.1998
(51) Int. Cl.: A61L 27/00

(54) **BIOARTIFIZIELLES TRANSPLANTAT UND VERFAHREN ZU SEINER HERSTELLUNG**
BIOSYNTHETIC TRANSPLANT AND METHOD FOR THE PRODUCTION THEREOF
TRANSPLANT BIOSYNTHETIQUE ET SON PROCEDE DE PRODUCTION

(30) Priorität: 27.06.1997 DE 19727497
(43) Veröffentlichungstag der Anmeldung: 05.04.2000
(73) Patentinhaber: Bader, Augustinus, 31275 Lehrte (DE); Steinhoff, Gustav, 31303 Burgdof (DE); Haverich, Axel, 30916 Isernhagen (DE)
(72) Erfinder: Bader, Augustinus, 31275 Lehrte (DE); Steinhoff, Gustav, 31303 Burgdof (DE); Haverich, Axel, 30916 Isernhagen (DE)
(74) Vertreter: Bösl, Raphael, Dr. rer. nat., Dipl.-Chem.
(86) Internationale Anmeldenummer: DE9801782
(87) Internationale Veröffentlichungsnummer: WO9900152

(56) Entgegenhaltungen:
- EP-A- 0 393 788
- EP-A- 0 564 786
- WO-A-95/24873
- WO-A-96/08213
- WO-A-96/32905
- US-A- 5 192 312

## Beschreibung

Die Erfindung betrifft ein Verfahren zur Herstellung eines bioartifiziellen Transplantats unter Verwendung einer zellfrei gemachten Bindegewebs-Matrix, die mit autologen Zellen des Transplantat-Empfängers oder mit gentechnisch modifizierten allogenen oder xenogenen Zellen besiedelt wurde. Ferner betrifft die Erfindung ein bioartifizielles empfängerspezifisches Transplantat, das nach dem genannten Verfahren erhältlich ist und aus bereichsweise mit verschieden differenzierten autologen Zellen besiedeltem interstitiellem Bindegewebe besteht.

In der Transplantationstechnik wird heute die Transplantation von Haut, Gefäßen und Organen chirurgisch bereits gut beherrscht und ist weit verbreitet. Die Bereitstellung geeigneter Transplantate ist jedoch noch immer schwierig. Die Transplantate kann man nach ihrer Art in drei große Gruppen einteilen. Zunächst gibt es Transplantate bzw. Implantate aus künstlichen Materialien, wie Kunststoffen, Metallen, Keramik, textilen Materialien usw. je nach Verwendung und dadurch sich ergebender Belastung.

Die synthetischen Implantate haben heute den Vorteil großer Haltbarkeit und sind besonders im orthopädischen Bereich durchgesetzt, da beispielsweise bei künstlichen Gelenken hohe Anforderungen an die Belastbarkeit des Materials gestellt werden. Künstliche Implantate haben jedoch vor allem den Nachteil, daß sie in keinem Falle mitwachsen und auch nicht wirklich einwachsen können. Durch letzteres entstehen häufig Probleme am Übergang vom künstlichen Implantat zu seiner natürlichen Umgebung.

Als zweites besteht die Möglichkeit, allogenes Material, z.B. Spenderorgane, oder u.U. auch xenogenes Material (tierischen Ursprungs) zu verwenden. Chemisch behandelte Transplantate tierischer Herkunft werden beispielsweise für den Herzklappenersatz beim Menschen verwendet. Das tierische Material wird dabei i.a. mit Glutaraldehyd behandelt, um die Strukturproteine zu stabilisieren und eine antigene Reaktion zu verhindern. Das mit Glutaraldehyd behandelte Gewebe unterliegt jedoch einer stetigen Verhärtung und fortschreitenden Calzifizierung nach der Transplantation. Diese Transplantate müssen daher alle paar Jahre ersetzt werden. Bei der Verwendung allogener Materialien, wie z.B. Spenderorganen ist eine ständige für den Organismus des Empfängers belastende Immunsuppression notwendig. Dennoch kann es zu Abstoßungsreaktionen aufgrund verschiedener Prozesse im Körper kommen.

Aus der WO-A-95 24873 ist ein Verfahren zur Erzeugung einer Hybrid-Bioprothese bekannt. Aus natürlichem, interstiellem Collagengewebe wurden native Zellen und potentielle immunologisch aktive lösliche Moleküle entfernt. Dann wurde es nacheinander mit extrazellulärem Matrix-Adhesionsfaktor, extrazellulärem Matrix-Glykosaminoglykan und Wachstumsfaktor, behandelt. Die Besiedelung erfolgt mit für den Empfänger allogenen oder autologen Fibroblasten.

Aus der WO-A-96 32905 ist ein Verfahren zur Behandlung von Körpergeweben, wie vaskuläre Strukturen, bekannt, bei dem ein für eine Implantation in einen menschlichen Wirt vorgesehenes Gewebe nacheinander einer hypotonischen/hypertonischen Behandlung und einer Lipase/Desoxyribonuclease-Behandlung unterzogen wird, um eine für eine Rezellularisierung geeignete Collagen und Elastin aufweisende Matrix zu erhalten.

In manchen Fällen wird schließlich versucht, körpereigenes Gewebe für eine Transplantation an anderer Stelle zu verwenden. Diese Methode wird beispielsweise bei Bypass-Operationen angewendet, wobei eine Vene von einer anderen Körperstelle entnommen und im Herzbereich eingesetzt wird, um mangelnde Druchblutung dort auszugleichen.

Auch bei Hautverpflanzungen wird häufig eigene, an anderer Stelle entnommene Haut verwendet. Bei der Verpflanzung von Haut besteht das größte Problem darin, daß praktisch nichts gewonnen wird, wenn die Hautfläche 1:1 verpflanzt wird, und daß demzufolge ein kleiner Hautbereich entnommen und anschließend expandiert werden muß. Diese Dehnung der entnommenen Haut ist jedoch für die Stabilität und Funktionalität der neu transplantierten Haut schädlich.

Es besteht daher ein großes Bedürfnis für Transplantat-Materialien oder fertig verwendbare Transplantate, die dem natürlichen Material soweit nachmodelliert sind, daß sie gut einwachsen, nach Möglichkeit zu keinen Abstoßungsreaktionen ("Host-Versus-Graft-reaction") führen, dadurch lange haltbar sind und vom Wirt wie körpereigenes Material aufgefaßt und im Rahmen des natürlichen Zellaustausches umgebaut werden, so daß möglichst sogar ein Mitwachsen der Transplantate im Körper des Empfängers ermöglicht wird.

Der Erfindung liegt daher die Problemstellung zugrunde, ein solches Transplantat und ein Verfahren zu seiner Herstellung zur Verfügung zu stellen.

Zur Lösung dieses Problems ist erfindungsgemäß ein Verfahren zur Herstellung eines bioartifiziellen Transplantats für einen ausgewählten Empfänger vorgesehen, welches die folgenden Schritte umfaßt:
a) Bereitstellung eines nativen, eine Collagen-Matrix enthaltenden allogenen oder xenogenen Gewebes bzw. Materials, Materials,
b) Entfernung weitgehend aller antigen-reaktiver Zellen aus der Collagen-Matrix mit Hilfe eines chemisch zellablösenden Mittels, das frei von Collagen angreifenden Enzymen ist,und anschließendes Spülen mit steriler Lösung, oder entsprechende mechanische Zellentfernung,
c) direkte Weiterverarbeitung des zellfrei gemachten, durch die Behandlung unter b) aufgelockerten Materials durch möglichst vollständige Besiedlung mit den jeweils gewünschten, autologen Zellen des Empfängers oder mit genetisch modifizierten für den Emfpänger angepaßten Zellen,wobei die Besiedelung des Materials örtlich mit verschiedenen differenzierten autologen Zellen erfolgt, wodurch ein unmittelbar einsetzbares Transplantat erhalten wird.

Aus der US-PS 5 336 616 ist bereits ein Verfahren zur Zubereitung eines Gewebes auf Collagen-Basis für die Transplantation bekannt, welches mit Hilfe der Schritte: chemische Vorbehandlung und Zellentfernung, Kryobehandlung, Trockenstabilisierung, Trocknung, Rehydrierung und Zellrekonstitution ein bioartifizielles Transplantat mit folgenden Eigenschaften ermöglichen soll: a) es enthält eine extrazelluläre Protein- und Collagen-Matrix, die möglicherweise vom Wirt remodelliert und repariert werden kann, b) es stellt eine intakte Membran-Grundlage für die erfolgreiche Wiederbesiedlung mit lebensfähigen Endotheloder Epithelzellen dar, c) es ruft primär keine Immunantwort beim Wirt hervor, d) es calzifiziert nicht und e) es kann einfach bei Raumtemperatur gelagert und transportiert werden. Das bekannte Verfahren beruht auf dem Konzept, daß als Transplantat eine möglichst "immun-neutrale" Stützgewebs-Matrix gebildet werden soll, die dann vom Transplantations-Empfänger im Körper umgebaut und remodelliert werden kann. Zur Erleichterung dieses Vorgangs ist auch die Möglichkeit anschließender Besiedlung des nach dem Verfahren hergestellten Transplantates vorgesehen.

Um zu vermeiden, daß die Matrix vom Körper des Transplantat-Empfängers als fremd erkannt wird, ist eine Präparation mit bestimmten Schritten vorgesehen. Zunächst wird das entnommene biologische Grundmaterial in eine Stabilisierungslösung eingelegt, um Strukturschäden zu vermeiden. Diese Stabilisierungslösung kann Antioxidantien, Quellmittel, Antibiotika, Protease-Inhibitoren und auch Relaxantien für glatte Muskulatur enthalten. Dann wird das vorbehandelte Gewebe in eine Azellularisierungslösung eingelegt, die im allgemeinen einen geeigneten Puffer, Salz, ein Antibiotikum, ein oder mehrere Detergentien, ein oder mehrere Protease-Inhibitoren und ein oder mehrere Enzyme enthält. Die so erhaltene azelluläre Matrix könnte nun mit einem vernetzenden Mittel, wie Glutaraldehyd, fixiert und bis zur Transplantation gelagert werden. Ansonsten wird sie einer üblichen Kryobehandlung unterzogen. Im Laufe dieser Kryobehandlung kann das Produkt bereits steril verpackt werden. Das Gewebe wird dann bei niedriger Temperatur unter Vakuumbedingungen einer möglichst schonenden Gefriertrocknung unterzogen. Diese Gefriertrocknung erfolgt offenbar als zusätzliche Maßnahme zur Vermeidung von Rejektionen, da angeblich gefriergetrocknetes Material verglichen mit frischem oder cryokonserviertem Material weniger Rejektionen verursacht. Das Material soll dann vorzugsweise in diesem Zustand gelagert und transportiert werden. Vor einer Transplantation wird das Gewebe rehydriert. Die Rehydrierung kann in Salz- oder Ringer-Lösung erfolgen und ggf. Protease-Inhibitor(en) enthalten. Weitere Zusatzstoffe können enthalten sein, z.B. Diphosphonate zur Inhibierung alkaliner Phosphatase und Unterdrückung der Calzifizierung, weiterhin Mittel zur Anregung der Neovaskularisierung und Wirtszellen-Infiltration nach der Transplantation, oder die Rehydrierung kann auch in einem Vernetzungsmittel wie Glutaraldehyd durchgeführt werden. Als zusätzliche Maßnahme ist schließlich die Besiedlung mit immuntoleranten lebensfähigen Zellen in vitro oder in vivo (nach der Transplantation) vorgesehen.

Das zentrale Konzept dieses bekannten Verfahrens besteht darin, eine azelluläre, quasi "neutrale" Matrix zu erhalten, die gut eingebaut wird und keine Rejektionen hervorruft. Die Betonung liegt dabei auf einer "Neutralisierung" der Matrix durch unter Umständen zahlreiche chemische Behandlungsschritte mit Enzymen, Detergentien, Antibiotika, Protease-Inhibitoren usw.. Ein weiterer Schwerpunkt liegt auf der guten Lager- und Transportfähigkeit, die durch die Cryokonservierung und Trocknung erreicht wird, wobei gleichzeitig eine weitere "Neutralisierung" der Matrix stattfinden soll.

Das bekannte Verfahren - und damit auch das danach erhaltene Transplantat - weist jedoch noch gravierende Nachteile auf. So hat es sich gezeigt, daß die polymere Collagenmatrix auch bei schonendem Einfrieren und Trocknen Strukturveränderungen unter Reduzierung der mechanischen Stabilität und Elastizität erleiden muß, da in vitro-Versuche zeigen, daß die Zellen bei einer Wiederbesiedlung auf einer vorher getrockneten oder cryobehandelten Collagen-Matrix wesentlich schlechter wieder einwachsen. Die Möglichkeit einer Renaturalisierung des Transplantats im Empfängerkörper wird dadurch sehr behindert oder gegebenenfalls zunichte gemacht.

Ferner erscheint der Weg bedenklich, eine freiliegende "neutralisierte" Collagen-Matrix zu schaffen. Die Collagen-Matrix kann zwar in vitro mit zahlreichen Chemikalien behandelt werden, dies läßt sich jedoch im Körper des Empfängers nicht weiterführen. Eine Behandlung mit Glutaraldehyd wird zu den oben bei xenogenen Materialien schon beschriebenen Problemen zunehmender Verhärtung und evtl. doch zu einer Calzifizierung führen. Ferner besteht die Gefahr, daß eine freiliegende Collagen-Matrix letztendlich doch durch endogene Collagenasen angegriffen wird, so daß es zu Abstoßungsreaktionen kommen kann. Bei freiliegender, unvollständig oder falsch besiedelter Collagen-Matrix kann es zu vermehrter Granulozyteneinwanderung und zu einer Thrombozyten-Adhäsion und Leukozyten-Einwanderung kommen, wodurch schließlich eine Entzündungsreaktion entsteht und das Transplantat strukturell und funktionell verändert wird.

Die Erfindung verfolgt demgegenüber ein neues Konzept. Großer Wert wird auch hier auf die vollständige Azellularisierung, d.h. die vollständige Entfernung antigen-reaktiver Zellen und anderer antigener Gewebekomponenten aus der Zellmatrix gelegt. Die Collagen-Matrix wird dann jedoch nicht in weiteren Behandlungsschritten immunologisch "neutralisiert" oder in ihrer Struktur verändert.

Als Ausgangsmaterial dient bei der Erfindung ein allogenes oder xenogenes Material, das die Grundstruktur für das gewünschte Gewebe, Gefäß oder Organ zur Verfügung stellen soll. Im Einzelfall könnte hier auch ein autologes Material verwendet werden, das dem späteren Transplantat-Empfänger zuvor entnommen wurde. Erfindungsgemäß soll die Azellularisierung dieses Ausgangsmaterials ausschließlich durch schonende Zellentfernung und anschließendes, ggf. reichliches Spülen mit steriler wässriger Lösung erfolgen. Die schonende Zellentfernung kann entweder durch schonende enzymatische Zellverdauung, beispielsweise durch Einlegen des Gewebes bzw. Materials in Trypsin-Lösung, erfolgen oder alternativ mit Hilfe eines chemisch zellablösenden Mittels, vorzugsweise mit Hilfe eines ionenspezifischen Komplexbildners.

Im Falle der enzymatischen Zellablösung werden durch das Verdauungsenzym Bindungsstellen in der Verankerung der Zellen mit ihrer Umgebung gelöst. Dies geschieht vorzugsweise durch Einlegen des Gewebes bzw. Materials in Trypsin-Lösung. Dieser Trypsin-Lösung kann bei Bedarf EDTA, EGTA, Triton oder TNN zugesetzt sein. Durch Einstellung der Zeitdauer, während derer das Enzym auf das Gewebe einwirkt, wird das Maß der Abverdauung gesteuert und es wird vermieden, daß ein Angriff auf die Collagen-Matrix selbst erfolgt. Die Trypsinbehandlung bewirkt auch eine gute Auflockerung der zellfrei gemachten Matrix, so daß die Neubesiedlung erleichtert wird.

Alternativ wird ein chemisch zellablösendes Mittel verwendet, das auf beliebige andere chemische Weise die Zellen an ihrer Verankerung zur Collagen-Matrix löst. Vorzugsweise ist vorgesehen, daß ein ionenspezifischer Komplexbildner verwendet wird, der den Zellen essentielle Ionen entzieht und so eine Ablösung verursacht. Durch die Komplexierung z.B. von Calziumionen wird die Bindung der Zellen untereinander und die Zell-Matrixbindung über Integrine aufgehoben.

Als ionenspezifischer Komplexbildner kann beispielsweise das calziumspezifische EGTA (Ethylenglykol- bis-(2-aminoethylether)-tetraessigsäure) eingesetzt werden. EGTA wird vorzugsweise kurzzeitig und hochkonzentriert eingesetzt. Auch andere Komplex- bzw. Chelatbildner, wie EDTA (Ethylendiamintetraessigsäure), Citrat oder Ethylendiamin oder sonstige chemisch zellabtötende und ablösende Mittel, die die Collagen-Proteinstruktur nicht angreifen, können verwendet werden. So bieten sich weiterhin an : BAPTA/AM (ein zellpermeabler Calzium-Chelator) DMSO, Gadolinium, Desferrioxamin, Desferrithiocin, Hexadentat oder auch Aminocarboxylat.

Die vorgenannten Mittel können einzeln oder in Mischungen eingesetzt werden, sie können durch andere Zusätze ergänzt werden, wie z.B. durch Tenside, die die Ablösung der Zellen erleichtern können (z.B. TRITON®). Vorzugsweise ist das chemisch zellablösende Mittel frei von Enzymen, die bei längerer Einwirkung Collagen angreifen könnten, wie z.B. Trypsin. Durch die genannten Mittel werden die Zellen gleichzeitig abgetötet und abgelöst. Das Ablösen kann durch intensives Spülen oder eine entsprechende, die Zellen mechanisch abscherende Behandlung untersützt werden.

Ein Vorteil der chemisch-mechanischen Behandlung liegt darin, daß der Behandlungsschritt, in dem die Collagen-Matrix zellfrei gemacht wird, nur deutlich weniger Zeit in Anspruch zu nehmen braucht als bei einer Behandlung mit Trypsin. Hierdurch sinkt erstens die Gefahr, daß die Collagen-Matrix selbst angegriffen wird, und zweitens bleibt die Tertiärstruktur besser erhalten, wenn das Substrat nicht zu lange in Lösung gequollen wird. Ein weiterer Vorteil besteht darin, daß durch den Eingsatz "harter Chemikalien" Probleme durch u.U. kontaminierte Enzyme tierischen Ursprungs vermieden werden können. Ein weiterer Vorteii liegt schließlich darin, daß die Basalmembran intakt bleibt. Die Basalmembran stellt das direkte und gewebespezifische Adhäsionssubstrat für die Endothelzellen dar. Der Glycosilierungsgrad der Matrixproteine beeinflußt auch die Zellimigration und somit ein späteres Einwandern von Zellen. Die natürliche Form des Collagengerüstes in ihrer dreidimensionalen Verbindung mit anderen Matrixkomponenten und Integrinen kann daher durch die Erfindung erhalten werden.

Auf die beschriebene Weise wird eine aufgelockerte, jedoch in ihrer Sekundär- und Tertiärstruktur dem nativen polymerisierten Collagen noch weitgehend entsprechende Collagenstruktur erhalten, die für das Einwachsen neuer Zellen, nämlich autologer Zellen des Empfängers oder. falls dies im Einzelfall möglich ist, anderer immuntoleranter Zellen bestens vorbereitet ist.

Die zellfrei gemachte, aufgelockerte Matrix wird daher allenfalls kurz zwischengelagert oder sterilisiert (beispielsweise radioaktiv mit UV oder Protonen bestrahlt, oder mit Ethylenoxid begast), jedoch nicht chemisch weiterbehandelt, sondern prinzipiell sofort, möglichst vollständig mit den jeweils gewünschten örtlich mit verschieden differenzierten, i.a. mit für den Empfänger autologen Zellen in vitro besiedelt. Diese Besiedlung kann in einem normalen Kulturmedium erfolgen, dem ein Antibiotikum zugesetzt sein kann. Im Falle der besonders schonenden und schnell verlaufenden Zellablösung durch Komplexbildner kann ggf. eine zusätzliche chemische oder Kryo-Behandlung erfolgen, wenn dies aus besonderen Gründen notwendig erscheint.

Der Erfindung liegt die Erkenntnis zugrunde, daß eine spätere Abstoßungsreaktion erfolgreich bereits dadurch vermieden werden kann, daß eine vollständige, im Einzelfall auch mehrschichtige Besiedlung der weitestgehend azellularisierten Matrix mit immuntoleranten Zellen, d.h. im allgemeinen mit autologen Zellen des Empfängers, vorgenommen wird. Statt autologer Zellen können auch Zellen anderen Ursprungs verwendet werden, die genetisch so verändert wurden, daß sie für den Transplantat-Empfänger "verträglich" sind, d.h., vom Körper nicht mehr als "fremd" erkannt werden. Auf diese Weise wird dem Empfänger ein Transplantat zur Verfügung gestellt, das an allen zugänglichen Stellen mit autologen Zellen bedeckt ist und daher vom Körper des Empfängers nicht als fremd erkannt werden wird. Dieses Transplantat wird im Körper den üblichen Umbildungsmechanismen unterworfen werden, so daß es auf natürliche Weise remodelliert, erneuert und unter Zelldifferenzierung weiter angepaßt wird. Das Transplantat ist daher bestens vorbereitet, besonders gut einzuwachsen, umgebaut zu werden und ggf. sogar mitzuwachsen. Durch die azellularisierte Matrix kann eine Vielzahl von Transplantatformen vorgegeben werden. Diese können je nach Anwendungszweck mit verschiedenen autologen Zellen besiedelt werden, und zwar ggf. auch bereichsweise mit unterschiedlichen autologen Zellen.

In Weiterbildung der Erfindung ist vorgesehen, daß in die Matrix Wachstumsfaktoren mit eingebracht werden. Hierbei kann es sich vorzugsweise um für die jeweiligen Zellen spezifische Faktoren, wie HGF, VEGF, FGF, ECGF oder PDGF handeln. Ebenso können auch Matrixfaktoren wie z.B. Fibronektin oder chemotaktische Faktoren eingesetzt werden.

In einer bevorzugten Ausführungsform werden die Wachstumsfaktoren bei der Zellbesiedlung eingebracht, indem genetisch modifizierte Zellen verwendet werden, die mit Genen für geeignete Wachstumsfaktoren transformiert wurden, so daß eine wenigstens transiente Expression an Wachstumsfaktor(en) im Zeitraum nach der Transplantation erfolgt. Die temporäre Expression von Wachstumsfaktor(en) kann helfen, die Akzeptanz für das Transplantat zu erhöhen, indem Kapillarisationsprozesse verstärkt oder ausgelöst werden. Für die Transformation bzw. Transfektion der Zellen ist eine geeignete Methode auszuwählen. Es kann sich dabei um die Elektroporation, liposomalen Gentransfer, rezeptorvermittelte Endozytose, Proteincoating oder sonst ein geeignetes der bekannten und in der Literatur hinlänglich beschriebenen Verfahren handeln.

Alternativ kann der Wachstumsfaktor auch direkt in die extrazelluläre Matrix eingebracht werden. Dies könnte beispielsweise in mikroinkapsulierter Form oder durch geeignete Beschichtung geschehen. Hierdurch sollte der Wachstumsfaktor kurzfristig oder über einen bestimmten Zeitraum ab Implantation retardiert in dem Transplantat feigesetzt werden, so daß zeitweise eine örtlich hohe Konzentration an Wachstumsfaktor erzeugt wird, von der eine Signalwirkung für die Auslösung von Kapillarisationsprozessen ausgeht.

Das nach dem Verfahren erhaltene Transplantat ist unmittelbar einsetzbar. Lagerung und Transport sollte höchst schonend unter sterilen und nicht dehydrierenden Bedingungen erfolgen. Eine gesonderte De- und Rehydrierung oder sonstige strukturverändernde Maßnahmen empfehlen sich an dem fertigen Transplantat nicht.

Insbesondere bei pareanchymatösen Organen ist die Steuerung, welche Zellen wo aufwachsen sollen, besonders wichtig, damit das Gewebe nicht als fremd erkannt wird. Bei röhrenförmigen Gefäßen und Herzklappen-Transplantaten werden zweckmäßigerweise außen autologe (Myo-)Fibroblasten und innen Endothelzellen verwendet. Die Fibroblasten wachsen mehrschichtig auf und sichern auf diese Weise eine äußerlich intakte Besiedlung mit autologen-Zellen, wodurch die Infiltration mit Granulationsgewebe veroder zumindest stark gehindert wird. Das Aufbringen der Zellen auf die Außenseite röhrenförmiger Gefäße kann z.B. mit Hilfe viskoser Flüssigkeiten erfolgen. Die Zellen halten sich dadurch besser auf der äußeren Oberfläche, so daß das Einwachsen erleichtert wird. Es kann eine viskose oder sogar gelierfähige Lösung Verwendung finden, die z.B. Collagen, Plasma oder Fibrinogen enthalten kann.

Die Erfindung umfaßt dementsprechend insbesondere auch bioartifizielle empfängerspezifische Transplantate, welche aus bereichsweise mit verschieden differenzierten autologen Zellen besiedeltem interstitiellem Bindegewebe bestehen. Anstelle von autologen Zellen, die vorher vom Transplantat-Empfänger gewonnen wurden, können auch quasi-autologe Zellen anderen Ursprungs eingesetzt werden, die gentechnisch im Hinblick auf den Empfänger verändert wurden. Die Collagen-Matrix des interstitiellen Bindegewebes soll nach Möglichkeit nirgends mehr freiliegen.

Handelt es sich bei dem erfidungsgemäßen Transplantat um ein Haut-Transplantat, ist es vorzugsweise außen (auf der körperabgewandten Seite) mit Keratinozyten und innen (körperseitig) mit Zellen mesodermalen Ursprungs besiedelt. Auch hierbei sind die Oberflächen jeweils vollständig besiedelt, so daß dort überwiegend keine Collagen-Matrix freiliegt, die auf die Dauer verhärten oder vom Körper als fremd erkannt und deshalb bekämpft werden könnte.

Neben Hauttransplantaten können auch bioartifizielle Herzklappen, Aorten, sonstige Gefäße, Sehnen, Cornea, Knorpel, Knochen, Larynx, Herz, Trachea, Nerven, Miniskus, Diskus intervertebralis oder z.B. Ureteren, Urethra und Blase erhalten werden. Letztere können zur Defektdeckung bei Patienten nach Operationen oder bei angeborenen Mißbildungen (z.B. Hypospadie) verwendet werden.

Das polymerisierte, praktisch im nativen Zustand belassene Collagen der Transplantat-Matrix ist von einer dem natürlichen Zustand weitgehend entsprechenden hohen mechanischen Stabilität und Elastizität. Dies ermöglicht, daß das bioartifizielle Transplantat lange, möglicherweise dauerhaft, ohne erneuten operativen Ersatz im Körper des Empfängers verbleiben kann. Darüberhinaus beeinflußt dieses Collagen die Zelldifferenzierung beim körpereigenen Umbau positiv. Das transplantierte artifizielle Organ wird leichter durch "inneren Umbau" körpereigen gemacht, d.h. daß die autologen Zellen die xenogene Matrix innerhalb des Organismus resorbieren und durch neue autologe Matrix ersetzen.

Im folgenden wird die Erfindung anhand von Beispielen beschrieben, die Isolationsprotokolle für die die Isolation autologer Zellen und Beispiele für die Durchführung des Verfahres umfassen.

### Beispiele:

### Herstellung eines bioartifiziellen Transplantats (allgemein)

### Gewebevorbereitung (Zellentfernung) mit Trypsin:

Das Gewebe wird nach der Entnahme in eine bevorzugt 0,05%ige Trypsinlösung gelegt und dort je nach Wandstärke des Materials z.B. 24, 48, 72 oder 96 Stunden belassen. Alternativ kann statt der Zeit die Konzentration der Trypsin-Lösung variiert werden. Das Gewebe soll vollständig mit Trypsinlösung bedeckt sein. Die Flüssigkeit wird vorzugsweise ständig durch Rühren in Bewegung gehalten. Die Temperatur sollte zwischen 25 und 37°C liegen.

Insbesondere bei sehr festem Gewebe kann zur Unterstützung der Zellablösung zusätzlich eine Veränderung des pH-Wertes oder eine schonende Ultraschall-Behandlung vorgenommen werden.

### Gewebevorbereitung (Zellentfernung) mit Komplex- bzw. Chelatbildner:

Typischerweise erfolgt die Behandlung des Gewebes durch Immersion in einer 1% EDTA isotonischen Kochsalzlösung bei 4°C für ca. 3 Stunden. Die Behandlungsdauer ist von der Konzentration des Komplexbildners abhängig. Bedarfsweise kann auch hier eine Unterstützung der Ablösung, beispielsweise duch Ultraschall, erfolgen.

Nach Abschluß der enzymatischen oder komplexierenden Ablösung von Zellen und antigenen Gewebeteilchen wird das Gewebe gespült d.h. mehrfach in Spüllösung eingelegt oder längere Zeit unter Durchfluß von Spüllösung gereinigt. Zum Spülen kann sterile wässrige Lösung wie z.B. NaCl-Lösung, PBS oder andere verwendet werden. Das Spülen kann mehrere Tage dauern und führt zusätzlich zu einer Entfernung vormals nicht abgeschwemmter Zellkörper.

Wenn die Behandlung mit Komplexbildner bei 4°C vorgenommen wurde, kann auch das Spülen vorteilhaft bei dieser Temperatur erfolgen. Dabei sollte wenigstens ca. 2 Stunden gespült werden.

Falls erforderlich, wird das Gewebe nun in isotonischer Lösung bei Kühlung auf etwa 4°C unter Zusatz von Antibiotika bis zur weiteren Besiedlung steril gelagert.

An dieser Stelle ist eine radioaktive Bestrahlung, beispielsweise eine γ-Sterilisierung, eine Begasung mit Ethylenoxid, eine UV- oder Protonenbestrahlung möglich.

Bei der Isolation der für die Besiedlung zu verwendenden Zellen (z.B. Endothelien glatte Muskelzellen, Fibroblasten) können konventionelle Isolationsprtokolle verwendet werden.

Die Besiedlung erfolgt bei 37°C unter Sterilbedingungen in einem Medium, dem ggf. Wachstumsfaktor (ECGF) zugesetzt sein kann. Für eine vollständige Besiedlung ist es vorteilhaft, daß Kulturmedium ständig zu bewegen und das bewegte Material häufig mit Sauerstoff in Kontakt zu bringen.

Die Besiedlung mit verschiedenartigen Zellen bzw. Zellpopulationen kann auf unterschiedliche Weise erfolgen.

Ein Vorteil der Erfindung ist, daß die Zellen eine extrazelluläre Matrix in einer weitestgehend der natürlichen Zusammensetzung entsprechenden Form und 3-D-Geometrie vorfinden. Das heißt, daß unter Vermeidung strukturschädlicher Prozesse, wie z.B. Dehydrierung und Rehydrierung, eine dem physiologischen Zustand nahekommende extrazelluläre Matrix für die Zellbesiedlung verwendet werden kann. Diese Matrix kann zeitlich gestaffelt besiedelt werden.

Sofern es sich um Haut handelt, kann das Gewebe zwischenzeitlich auf einem Träger fixiert oder in einem Rahmen eingespannt werden, wonach dann nur die eine Seite mit Zellen bespült wird. Dieses Verfahren kann für die Ober- und Unterseite angewendet werden.

Auch andere Materialien können durch geeignetes Einspannen und/oder Abdichten bestimmter Bereiche gezielt besiedelt werden. Beispielsweise kann von oben mit Keratinozyten und von unten mit Bindegewebszellen und ggf. Zellen von Hautanhangsgebilden besiedelt werden. Alternativ können von oben zeitlich vor der Besiedlung mit Keratinozyten Zellen von Hautanhangsgebilden aufgegeben werden.

Röhrenförmige Transplantate können z.B. zunächst innen im Durchfluß besiedelt werden, dann abgebunden oder eingespannt und außen besiedelt werden. Bei der Herstellung eines bioartifiziellen Gefäßes ist es beispielsweise möglich, von außen Myofibroblasten und innerhalb des Lumens humane Endothelien aufzugeben. Ziel ist es, das Einwachsen der Zellen in die Wand des Gefäßes und eine Differenzierung der Zellen am Migrationsziel innerhalb der 3D-Mikroumgebung der extrazellulären Matrix zu erreichen. Diese Einwanderung der Zellen in die Matrix stellt ein wesentliches Merkmal und gleichzeitig einen bedeutenden Vorteil der Erfindung dar.

Die Gefäßbesiedlung von außen kann auch zunächst mit glatten Muskelzellen und danach mit (Myo-)Fibroblasten erfolgen.

Alternativ können innen, in das Lumen, zunächst Myofibroblasten gegeben werden und danach - zeitlich so versetzt, daß sich eine konfluente Myofibroblasten-Zellschicht gebildet hat - ebenfalls in das Lumen Endothelien (in Kulturmedium suspendiert).

Die Gewinnung der Endothelien und glatten Muskelzellen wird entsprechend dem Stand der Technik durchgeführt.

### GEWEBEGWINNUNG

Vena saphena-Stücke des peripheren Beins von Patienten von ca. 1 cm Länge werden in heparinisiertes Vollblut gegeben und auf diese Weise in das Labor transportiert. Dort erfolgt die Isolation der Endothelien und Myofibroblasten für die weitere Expansion in vitro.

### ISOLATION UND KULTUR VON ENDOTHELIEN, GLATTEN MUSKELZELLEN UND FIBROBLASTEN

### 1. Materialien

- HBSS (Clintec, Salvia, Homburg)
- PBS, darin CA²⁺ und Mg²⁺ (Sigma)
- Collagenase A (Boehringer Mannheim)
- M-199 mit L-Glutamin (Sigma) , darin 10% (20%) FBS (Sigma)
- gepooltes Humanserum
- 100 u/ml Penicillin (Sigma), 100 µg/ml Streptomycin (Sigma)
- 5000 u/ml Heparin (Heparin Novo, Nordisk, Mainz, frei von Konservierungsstoffen)
- 6-well Kulturplatten (Greiner, Frickenhausen, Deutschland)
- Fibronectin Beschichtung

### 2. Endothelzellen

### 2.1. Beispiel für eine Zellgewinnung

Die Zellgewinnung erfolgt durch Füllen des Segments mit PBS, darin Ca²⁺ und Mg²⁺ (Sigma) und 0,2% Collagenase A (Boehringer, Mannheim) (alternativ HBSS oder M-199 mit 0,2% Collagenase A), 30 min Inkubieren in HBSS (5% CO₂/95% Luft/37°C), evtl. Verlängerung der Inkubationszeit auf 1h, Fushen mit 50 ml M-199 mit L-Glutamin (Sigma), darin 20% FBS (Sigma) (alternativ: Aufschneiden des Gefäßes und Abschaben der Zellen in einer mit Medium gefüllten Petrischale), Auffangen der Zellen und Zentrifugieren (10 min bei 300 xg), Resuspendieren in 5 ml M-199, darin 20% FKS, 10u/ml Penicillin (Sigma), 100µg/ml Strepromycin (Sigma), 50µg/ml EGF (endothilial growth factor) (Boehringer, Mannheim), 5000 u/ml Heparin und Kultivieren auf mit 1%iger Gelatine vorbeschichteten 6-well Kulturplatten. Alternativ kann anstelle von Humanserum auch FBS oder NCS verwendet werden.

### 2.2 Kultur von Endothelzellen

Die Kultur kann erfolgen durch: Inkubation bei 5% CO₂, 95% Luft bei 37°C unter Mediumwechsel alle 3 Tage. Nach Erreichen der Konfluenz wird zur Ablösung der Zellen eine Lösung mit 0,05% Trypsin und 0,02 % EDTA (Sigma) in PBS ohne Ca²⁺ und Mg²⁺ verwendet (Inkubation 2 bis 3 Minuten bei Raumtemperatur). Alternativ kann eine mechanische Trennung durch "Abschaben" erfolgen. Anschließend wird M-199, darin 20% FBS (zur Trypsininaktivierung) gewaschen. Es wird 10 min bei 300 x g zentrifugiert und anschließend resuspendiert. Schließlich wird in 75cm²-Kultuflaschen subkultiviert (1. Passage, 2. Passage in 175cm²-Flaschen über ca. 2 Wochen). Diese Zellen werden zur Aussaat auf das Transplantat verwendet.

### 3. Myofibroblasten

### 3.1. Gewinnung von SMC ("smooth muscle cells" = glatte Muskelzellen) und FB (fibroblasts, Fibroblasten)

Die Gewinnung erfolgt in folgenden Schritten:
- mechanische Abtrennung der Adventitia (FB) vom deendothelialisierten Gefäß
- Zerkleinerung in 1mm-Stücke
- die Stücke werden mit wenig Medium in eine Kulturflasche gegeben
- nach 2-3 Tagen kleben die Gewebeteile auf dem Flaschenboden
- die Kulturflaschen werden täglich für 8 h senkrecht gestellt.

### 3.2 Kultivierung von SMC und FB

Die Kutivierung erfolgt mit einer Zelldichte von 10000 Zellen/cm² bei 37°C, 95% Luft und 5% CO₂ bei einem Mediumwechsel alle 2 bis 3 Tage bis zur weitgehenden Konfluenz; dann Typsinierung und Passagierung.

Zu den Figuren:

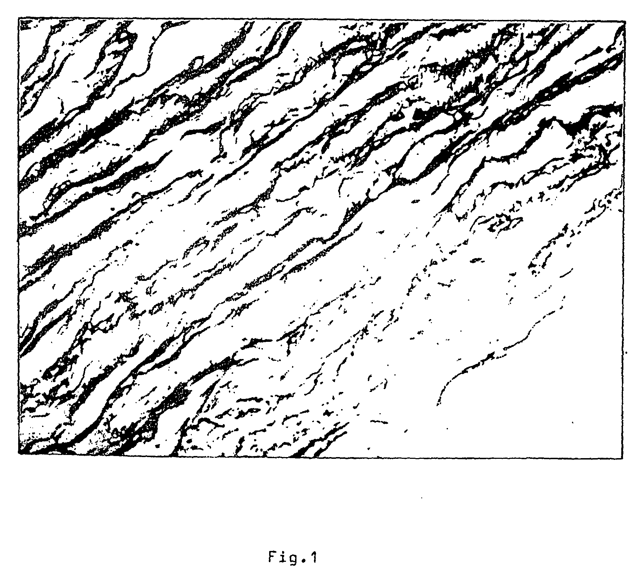

Figur 1: Aorta des Schweins nach Behandlung (Versuch entsprechend Schritten a) und b) des erfindungsgemäßen Verfahrens). Die Matrix ist aufgelockert und frei von Zellen. Die Zellentfernung erfolgte hier mit Trypsin.

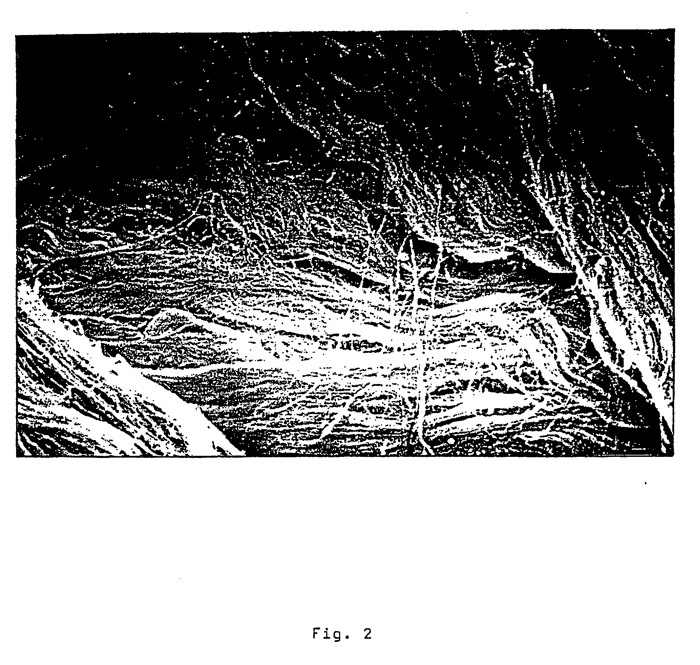

Figur 2: Rasterelektronische Aufnahme der Oberfläche einer mit Trypsin entsprechend der Erfindung azellularisierten Oberfläche einer Aortenwand. Die Matrixfibrillen sind dargestellt.

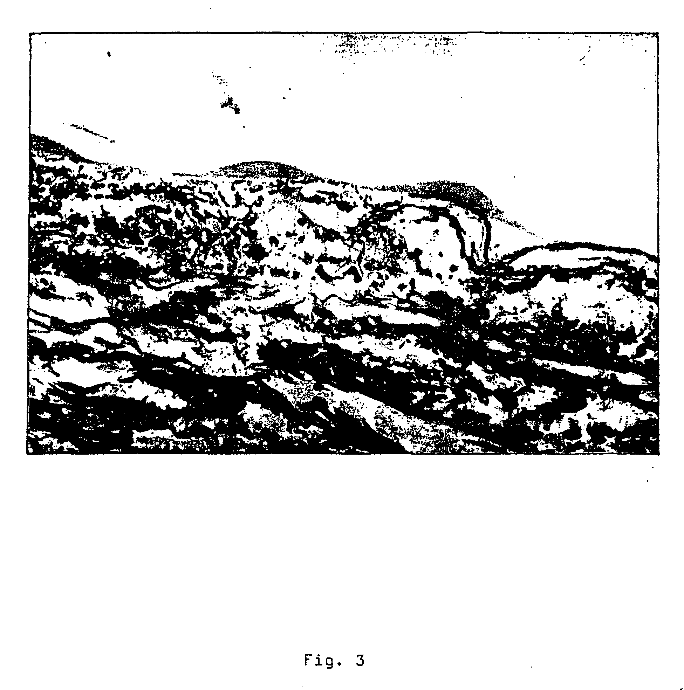

Figur 3: Aorta des Schweins nach Besiedlung mit Endothelien, die einen Monolayer an der oberflächlichen Lumenseite bilden.

## Patentansprüche

1. Verfahren zur Herstellung eines bioartifiziellen Transplantats für einen ausgewählten Empfänger, welches die folgenden Schritte umfaßt:
a) Bereitstellung eines nativen, eine Collagen-Matrix enthaltenden allogenen oder xenogenen Gewebes bzw. Materials,
b) Entfernung weitgehend aller antigen-reaktiver Zellen aus der Collagen-Matrix mit Hilfe eines chemisch zellablösenden Mittels, das frei von Collagen angreifenden Enzymen ist, und anschließendes Spülen mit steriler wässriger Lösung, oder entsprechende mechanische Zellentfernung,
c) direkte Weiterverarbeitung des zellfrei gemachten, durch die Behandlung unter b) aufgelockerten Materials durch möglichst vollständige Besiedlung mit den jeweils gewünschten autologen Zellen des Empfängers oder mit genetisch modifizierten für den Empfänger angepaßten Zellen, wobei die Besiedlung des Materials örtlich mit verschiedenen differenzierten autologen Zellen erfolgt, wodurch ein unmittelbar einsatzbereites Transplantat erhalten wird.

2. Verfahren nach Anspruch 1, **dadurch gekennzeichnet, daß** das zu besiedelnde Material ein röhrenförmiges Gefäß ist, das außen mit Myofibroblasten und innen mit Endothelzellen besiedelt wird.

3. Verfahren nach Anspruch 2, **dadurch gekennzeichnet, daß** das röhrenförmige Gefäß außen zunächst mit glatten Muskelzellen und danach mit Myofibroblasten und innen zunächst mit Myofibroblasten und danach mit Endothelien besiedelt wird.

4. Verfahren nach Anspruch 1, **dadurch gekennzeichnet, daß** das zu besiedelende Material eine sich flach ausdehnende, als Hauttransplantat vorgesehene Collagen-Matrix ist, die von oben mit Keratinozyten und von unten mit Bindegewebszellen besiedelt wird.

5. Verfahren nach Anspruch 4, **dadurch gekennzeichnet, daß** die Matrix von unten mit Bindegewebszellen und Zellen von Hautanhangsgebilden besiedelt wird.

6. Verfahren nach Anspruch 4 oder 5, **dadurch gekennzeichnet, daß** der Matrix von oben zeitlich vor der Besiedelung mit Keratinozyten Zellen von Hautanhangsgebilden aufgegeben werden.

7. Verfahren nach einem der Ansprüche 1 bis 6, **dadurch gekennzeichnet, daß** die schonende Zellentfernung durch eine enzymatische Zellverdauung geschieht, vorzugsweise durch Einlegen des Gewebes oder Materials in Trypsin-Lösung.

8. Verfahren nach einem der Ansprüche 1 bis 7, **dadurch gekennzeichnet, daß** die schonende Zellentfernung mit Hilfe eines chemisch zellablösenden Mittels erfolgt, vorzugsweise mit Hilfe eines ionenspezifischen Komplexbildners, insbesondere EDTA oder EGTA.

9. Verfahren nach einem der Ansprüche 1 bis 8, **dadurch gekennzeichnet, daß** das Material zwischen Schritt a) und b) undloder zwischen Schritt b) und c) in isotonischer Lösung bei Kühlung auf vorzugsweise 4 °C und unter Zusatz von Antibiotika steril zwischengelagert und/oder gegebenenfalls schonend sterilisiert wird, vorzugsweise durch Begasen oder Bestrahlen, insbesondere mit γ-Strahlen, UV oder Protonen.

10. Verfahren nach Anspruch 2, **dadurch gekennzeichnet, daß** das Aufbringen der Zellen auf die Außenseite röhrenförmiger Gefäße mit Hilfe viskoser Flüssigkeiten erfolgt.

11. Verfahren nach einem der Ansprüche 1 bis 10, **dadurch gekennzeichnet, daß** in die Matrix zusätzlich wenigstens ein Wachstumsfaktor, Matrixfaktor oder chemotaktischer Faktor eingebracht wird - vorzugsweise mit Hilfe der Zellen bei der Besiedelung, oder vor der Besiedelung durch Einbringen des Wachstumsfaktors in die azellularisierte Matrix, oder nach der Besiedelung durch Einbringen des Wachstumsfaktors in die besiedelte Matrix.

12. Verfahren nach Anspruch 11, **dadurch gekennzeichnet, daß** für die Besiedelung genetisch modifizierte Zellen verwendet werden, die mit Wachstumsfaktor(en) codierenden Genen transformierte oder transfiziert sind.

13. Verfahren nach einem der Ansprüche 1 bis 12, **dadurch gekennzeichnet, daß** das erhaltene Transplantat für Lagerung und Transport steril unter dehydrierenden Bedingungen gelagert wird.

14. Verfahren nach einem der Ansprüche 1 bis 13, **dadurch gekennzeichnet, daß** das collagenhaltige allogene oder xenogene Gewebe oder Material Herzklappen, Haut, Gefäße, Aorten, Sehnen, Comea, Knorpel, Knochen, Trachea, Nerven, Miniskus, Diskus intervertebralis, Ureteren, Urethra und Blase umfaßt.

15. Bioartifizielles empfängerspezifisches Transplantat, welches aus bereichsweise mit verschieden differenzierten autologen oder gentechnisch modifizierten für den Empfänger angepaßten Zellen besiedeltem interstitiellem Bindegewebe besteht, insbesondere erhältlich durch ein Verfahren nach einem der Ansprüche 1 bis 14.

16. Transplantat nach Anspruch 15, **dadurch gekennzeichnet, daß** es sich um ein parenchymatöses Organ handelt.

17. Transplantat nach Anspruch 15, **dadurch gekennzeichnet, daß** es sich um eine bioartifizielle Herzklappe handelt, welche außen mehrschichtig mit Myofibroblasten und innen mit Endothelzellen besiedelt ist, wobei die Oberflächen jeweils vollständig besiedelt sind, so daß dort weitgehend keine Collagen-Matrix freiliegt.

18. Transplantat nach Anspruch 15, **dadurch gekennzeichnet, daß** es sich um ein Hauttransplantat handelt, welches außen (auf der körperabgewandten Seite) mit Keratinozyten und innen (körperseitig) mit Zellen mesodermalen Ursprungs besiedelt ist, wobei die Oberflächen jeweils vollständig besiedelt sind, so daß dort überwiegend keine Collagen-Matrix freiliegt.

## Claims

1. Process for the preparation of a bioartificial transplant for a selected recipient, which comprises the following steps:
a) provision of a native allogenic or xenogenic tissue or material containing a collagen matrix,
b) removal of substantially all antigen-reactive cells from the collagen matrix with the aid of a chemical cell-detaching agent which is free of enzymes attacking collagen, and subsequent rinsing with sterile aqueous solution, or corresponding mechanical cell removal,
c) direct reprocessing of the material which is rendered cell-free and loosened by the treatment under b) by as complete as possible colonization with the autologous cells of the recipient which are desired in each case or with genetically modified cells suited to the recipient, the colonization of the material being carried out locally with various differentiated autologous cells, by means of which an immediately ready-for-use transplant is obtained.

2. Process according to Claim 1, **characterized in that** the material to be colonized is a tubular vessel which is colonized outside with myofibroblasts and inside with endothelial cells.

3. Process according to Claim 2, **characterized in that** the tubular vessel is colonized outside first with smooth muscle cells and then with myofibroblasts and inside first with myofibroblasts and then with endothelial cells.

4. Process according to Claim 1, **characterized in that** the material to be colonized is a collagen matrix extending flatly and intended as a skin transplant, which is colonized from above with keratinocytes and from below with connective tissue cells.

5. Process according to Claim 4, **characterized in that** the matrix is colonized from below with connective tissue cells and cells of skin appendages.

6. Process according to Claim 4 or 5, **characterized in that** the cells of skin appendages are placed on the matrix from above prior to the colonization with keratinocytes.

7. Process according to one of Claims 1 to 7, **characterized in that** the gentle cell removal takes place by means of an enzymatic cell digestion, preferably by putting the tissue or material into trypsin solution.

8. Process according to one of Claims 1 to 7, **characterized in that** the gentle cell removal is carried out with the aid of a chemical cell-detaching agent, preferably with the aid of an ion-specific complexing agent, in particular EDTA or EGTA.

9. Process according to one of Claims 1 to 8, **characterized in that** the material is intermediately stored aseptically between steps a) and b) and/or between steps b) and c) in isotonic solution with cooling to preferably 4°C and with addition of antibiotics and/or optionally gently sterilized, preferably by treatment with gas or irradiation, in particular with γ-rays, UV or protons.

10. Process according to Claim 2, **characterized in that** the application of the cells to the outside of tubular vessels is carried out with the aid of viscous liquids.

11. Process according to one of Claims 1 to 10, **characterized in that** at least one growth factor, matrix factor or chemotactic factor is additionally introduced into the matrix - preferably with the aid of the cells during colonization or before colonization by introducing the growth factor into the acellularized matrix, or after colonization by introducing the growth factor into the colonized matrix.

12. Process according to Claim 11, **characterized in that**, for colonization, genetically modified cells are used which have been transformed or transfected with genes encoding growth factor(s).

13. Process according to any one of Claims 1 to 12, **characterized in that** the transplant obtained is stored aseptically under dehydrating conditions for storage and transport.

14. Process according to one of Claims 1 to 13, **characterized in that** the collagen-containing allogenic or xenogenic tissue or material comprises heart valves, skin, vessels, aortas, tendons, cornea, cartilage, bone, trachea, nerves, meniscus, intervertebral disk, ureters, urethra and bladder.

15. Bioartificial recipient-specific transplant which consists of interstitial connective tissue colonized regionally with various differentiated autologous or genetically modified cells suited to the recipient, in particular obtainable by a process according to one of Claims 1 to 14.

16. Transplant according to Claim 15, **characterized in that** it is a parenchymatous organ.

17. Transplant according to Claim 15, **characterized in that** it is a bioartificial heart valve which outside is colonized in multilayered form with myofibroblasts and inside with endothelial cells, the surfaces in each case being completely colonized such that substantially no collagen matrix is exposed there.

18. Transplant according to Claim 15, **characterized in that** it is a skin transplant which is colonized outside (on the side facing away from the body) with keratinocytes and inside (body side) with cells of mesodermal origin, the surfaces in each case being completely colonized such that substantially no collagen matrix is exposed there.

## Revendications

1. Procédé pour fabriquer un transplant bioartificiel pour un receveur sélectionné, comprenant les étapes suivantes:
a) préparation d'un tissu ou d'un matériau allogène ou xénogène natif, contenant une matrice de collagène,
b) retrait dans une large mesure de toutes les cellules réactives à l'antigène, à partir de la matrice de collagène à l'aide d'un milieu réalisant une dissolution chimique des cellules et qui est libre d'enzymes agressifs pour la collagène, puis lavage avec une solution aqueuse stérile, ou élimination mécanique correspondante des cellules,
c) poursuite directe du traitement de la matière débarrassée des cellules, qui est agrégée par le traitement exécuté en b), au moyen d'un peuplement aussi complet que possible avec des cellules autologues respectivement désirées du receveur ou avec des cellules modifiées génétiquement adaptées au receveur, le peuplement de la matière s'effectuant localement avec les différentes cellules autologues différenciées, ce qui permet d'obtenir un transplant prêt à être directement utilisé.

2. Procédé selon la revendication 1, **caractérisé en ce que** la matière à peupler est un vaisseau de forme tubulaire, qui est peuplé extérieurement de myofibroblastes et intérieurement par des cellules endothéliales.

3. Procédé selon la revendication 2, **caractérisé en ce que** le vaisseau de forme tubulaire est peuplé extérieurement tout d'abord par des cellules musculaires lisses, puis par des myofibroblastes et intérieurement tout d'abord par des myofibroblastes, puis avec de l'endothélium.

4. Procédé selon la revendication 1, **caractérisé en ce que** la matière à peupler est une matrice de collagène qui s'étend à plat, est prévue en tant que transplant de peau et est peuplée à partir du haut par des kératinocytes et à partir du bas par des cellules conjonctives.

5. Procédé selon la revendication 4, **caractérisé en ce que** la matrice est peuplée à partir du bas par des cellules conjonctives et par des cellules phanères.

6. Procédé selon la revendication 4 ou 5, **caractérisé en ce qu'**avant le peuplement avec des kératinocytes, des phanères sont ajoutés à partir du haut à la matrice.

7. Procédé selon l'une des revendications 1 à 6, **caractérisé en ce que** l'élimination des cellules avec ménagements est réalisée au moyen d'une digestion enzymatique des cellules, de préférence par insertion du tissu ou de la matière dans une solution de trypsine.

8. Procédé selon l'une des revendications 1 à 7, **caractérisé en ce que** l'élimination des cellules avec ménagements est réalisée à l'aide d'un agent réalisant une dissolution chimique des cellules, de préférence à l'aide d'un agent complexant spécifique du point de vue ionique, notamment du EDTA ou du EGTA.

9. Procédé selon l'une des revendications 1 à 8, **caractérisé en ce qu'**entre les étapes a) et b) et/ou entre les étapes b) et c), la matière est stockée temporairement de façon stérile dans une solution isotonique avec refroidissement de préférence à 4°C moyennant l'addition d'antibiotiques et/ou est éventuellement stérilisée avec ménagements, de préférence par action d'un gaz ou d'un rayonnement, notamment avec un rayonnement γ, des ultraviolets ou des protons.

10. Procédé selon la revendication 2, **caractérisé en ce que** l'application des cellules sur la face extérieure de vaisseaux de forme tubulaire s'effectue à l'aide de liquides visqueux.

11. Procédé selon l'une des revendications 1 à 10, **caractérisé en ce que** dans la matrice on introduit en outre un facteur de croissance, un facteur matriciel ou un facteur chimiotactique - de préférence à l'aide des cellules lors du peuplement, ou avant le peuplement, par introduction du facteur de croissance dans la matrice, dont les cellules sont éliminées, ou après le peuplement par introduction du facteur de croissance dans la matrice peuplée.

12. Procédé selon la revendication 11, **caractérisé en ce que** pour le peuplement on utilise des cellules modifiées génétiquement, qui sont transformées ou transfectées avec un ou des facteurs de croissance avec des gènes, qui codent un ou des facteurs de croissance.

13. Procédé selon l'une des revendications 1 à 12, **caractérisé en ce que** le transplant obtenu est rangé, pour son stockage et son transfert, de façon stérile dans des conditions réalisant une déshydratation.

14. Procédé selon l'une des revendications 1 à 13, **caractérisé en ce que** le tissu ou la matière halogène ou xénogène, contenant du collagène, comprend les valves cardiaques, la peau, des vaisseaux, l'aorte, des tendons, la cornée, du cartilage, la trachée, les nerfs, le ménisque, le disque intervertébral, l'uretère, l'urètre et la vessie.

15. Transplant bioartificiel spécifique au receveur, qui est constitué par un tissu conjonctif interstitiel peuplé par endroit avec différentes cellules différenciées autologues ou modifiées par la technique génétique, adaptées pour le receveur, notamment disponible au moyen d'un procédé selon l'une des revendications 1 à 14.

16. Transplant selon la revendication 15, **caractérisé en ce qu'**il s'agit d'un organe parenchymateux.

17. Transplant selon la revendication 15, **caractérisé en ce qu'**il s'agit d'une valve cardiaque bioartificielle, qui est peuplée extérieurement, sur plusieurs couches, par des miofibroblastes et intérieurement par des cellules endothéliales, les surfaces étant respectivement complètement peuplées de telle sorte que dans une large mesure aucune matrice de collagène n'est libérée en cet endroit.

18. Transplant selon la revendication 15, **caractérisé en ce qu'**il s'agit d'un transplant de la peau, qui est peuplé extérieurement (sur le côté tourné à l'opposé du corps) par des kératinocytes et intérieurement (du côté du corps) par des cellules d'origine mésodermale, les surfaces étant peuplées respectivement complètement de sorte que dans une large mesure aucune matrice de collagène n'est libérée en cet endroit.
